Europäisches Patentamt

(19) European Patent Office

Office européen des brevets

(11) Publication number: **0 223 742 B1**

## EUROPEAN PATENT SPECIFICATION

(12)

(45) Date of publication of patent specification:
23.01.91 Bulletin 91/04

(51) Int. Cl.$^5$: **G01N 25/08**

(21) Application number: 86830209.2

(22) Date of filing: 17.07.86

(54) **Apparatus and method for determining the boiling point of liquids, particularly for determining the boiling point of non-eutetic aqueous mixtures.**

(30) Priority: 10.10.85 IT 6785885

(43) Date of publication of application:
27.05.87 Bulletin 87/22

(45) Publication of the grant of the patent:
23.01.91 Bulletin 91/04

(84) Designated Contracting States:
AT BE CH DE FR GB IT LI LU NL SE

(56) References cited:
AT-B- 376 803
DE-A- 2 721 232
US-A- 4 484 823

(73) Proprietor: FIAT LUBRIFICANTI S.r.l.
Via Santena 1/3
I-10029 Villastellone (Torino) (IT)

(72) Inventor: Chiantelassa, Enrica c/o FIAT
LUBRIFICANTI
Via Santena 1
I-10029 Villastellone Torino (IT)
Inventor: Chiampo, Pietro c/o FIAT
LUBRIFICANTI
Via Santena 1
I-10029 Villastellone Torino (IT)

(74) Representative: Bosotti, Luciano et al
c/o Jacobacci-Casetta & Perani S.p.A. Via
Alfieri, 17
I-10121 Torino (IT)

Jouve, 18, rue Saint-Denis, 75001 PARIS

## Description

The present invention relates to the determination of the boiling point of liquids such as, for example, non-eutetic aqueous mixtures used in hydraulic braking circuits for motor vehicles.

With reference to these mixtures, in order to determine a conventional boiling point which is univocally defined, it is necessary to ensure that in making the measurement it is possible :

- to impose a predetermined heating gradient on the mixture,
- to determine the degree of reflux of the condensed liquid, and
- to cause boiling without loss of vapour to the external environment.

More specifically, the present invention relates to apparatus and methods having the characteristics set forth in the pre-characterising portions of claims 1 and 12, respectively, which correspond to the disclosure of e.g. AT-B 376 803, DE-A-2 721 232 or US-A-4 484 823.

The object of the present invention is to provide apparatus and a method which satisfy all the requirements indicated above.

According to the present invention, this object is achieved by a virtue of apparatus having the further characteristic set forth in claim 1.

In other words, apparatus with the characteristics specified above is able to carry out a method, such as called for in claim 12, which enables the boiling point of liquids to be determined and which comprises the steps of :

- heating a predetermined quantity of the liquid of whose boiling point it is wished to determine,
- collecting the vapours from the boiling liquid so as to form drops of condensation from the vapours,
- detecting the formation of the first drop of condensation, and
- measuring the temperature of the liquid on formation of the first drop of condensation.

In a preferred embodiment of the apparatus of the invention, the condenser element is applied so as to close the container with a substantially gas-tight seal. Preferably, the condenser element has ducts which put the container into communication with the external environment and the dimensions of which are selected so that the vapours from the boiling liquid in the container which enter the ducts condense completely within the ducts themselves.

The invention will now be described, purely by way of non-limiting example, with reference to the appended drawings, in which :

Figure 1 is a partially sectioned, partially cut-away side elevational view illustrating the structure of the apparatus according to the invention,

Figure 2 is a block schematic diagram showing the internal structure of one of the elements illustrated in the Figure, and

Figure 3 illustrates one possible way of carrying out the method of the invention, in the form of a flow diagram which regulates the operation of the block schematic diagram of Figure 2.

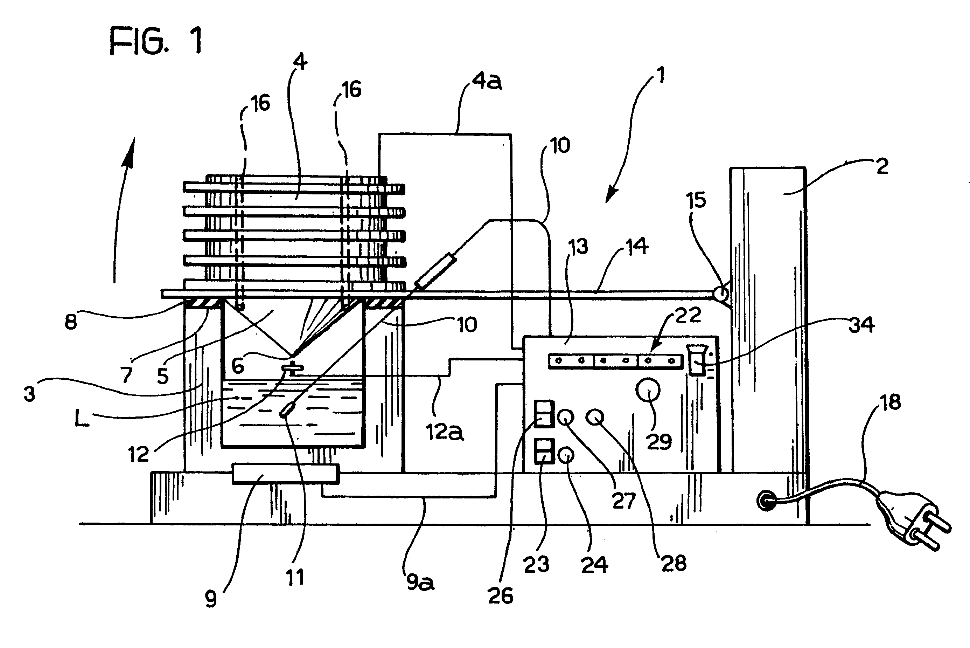

In Figure 1 apparatus for use, for example, in determining the boiling point of non-eutectic aqueous mixtures, such as those used as the operating fluid in hydraulic braking circuits for motor vehicles, is generally indicated 1.

"New" mixtures, that is, those which are at the beginning of their cycle of use, have very high boiling points, above 200°C. With time and with use, these mixtures tend to "age" and, in the presence of atmospheric humidity, absorb increasing quantities of water with a consequent lowering of the boiling point.

The lowering of this boiling point prejudices the efficiency and safety of the braking circuits. As a result of prolonged braking, the temperature of the mixture may in fact rise above the boiling point, gas bubbles developing within it and thus giving rise to the phenomenon normally known as "vapour lock".

The possibility of evaluating the boiling point in the braking circuit rapidly and precisely is thus essential in order to establish when the liquid is no longer suitable for use as a result of excessive lowering of its boiling point.

The apparatus 1 comprises, mounted in a frame or housing 2 which may, to advantage, take the form of a case or rack useable in an office or service station, the following components :

- a cup-shaped container 3, normally of refractory ceramic material, into which a predetermined quantity of a liquid L whose boiling point it is wished to detect may be introduced (for example, with a syringe),
- an air condenser 4 disposed so as to close the container 3 and constituted essentially by a metal (for example, aluminium) body including an upper cylindrical portion having circumferential heat-dissipating fins and a lower conical or generally pointed portion 5 having a vertex region 6 which extends into the beaker 3 ; an annular sealing washer 8 of insulating material is located on the free edge 7 of the container 3 and supports the condenser member 4 in the region of the junction between the upper part and the lower part 5 thereof,
- an element 9 for heating the liquid L constituted by a resistor disposed in correspondence with the bottom of the container 3,
- a temperature sensor 10 constituted essentially by a rod which extends into the container 3 and is provided with a detector probe 11 (constituted by a thermocouple, a resistor of a coefficient of resistance that varies with temperature (NTC or PTC), or by an equivalent device) disposed at a height equal to about half that of the level of filling of the liquid L in the container 3,

– an electrode 12 arranged to act as a hygrometric sensor located immediately adjacent the vertex 6 of the lower portion 5 of the condenser element 4, and

– an electrical control and operating circuit 13 the structure and configuration of the connections of which will be described more fully below.

Preferably, in order to facilitate the use of the apparatus 1 and to make it easier to maintain, the condenser element 4, the temperature sensor 10 and the hygrometric sensor 12 are mounted at one end of an elongate bracket member 14 the other end of which is connected by a hinge 15 to the frame 2.

Thus, the assembly formed by the condenser element 4 and the two sensors 10 and 12 may be pivoted upwardly, as shown schematically by the arrow in the left-hand part of Figure 1, so as to uncover the mouth of the container 3 and render it accessible from the exterior. The container 3 is itself releasably mounted in the frame 2. It may thus be removed from the frame 2 to be cleansed of any residues of the liquid subjected to the test.

Again with reference to the condenser element 4, several ducts (usually three staggered angularly by 120° around the periphery of the upper cylindrical part of the condenser 4) are indicated 16 and extend vertically through the body of the condenser element 4 so as to put the interior of the container 3 into communication with the exterior when the condenser element 4 is applied so as to close the container 3 itself. The presence of the annular sealing washer 8 and the fact that the weight of the condenser element 4 bears on the washer 8 with a pressure which can be increased further if desired by other resilient elements, not illustrated, means that the connection between the container 3 and the condenser element 4 around the mouth of the container 3 is vapour-tight. The purpose of this arrangement is to avoid loss of vapour to the atmosphere during boiling of the liquid within the container 3, since this could prejudice the correctness of the determination.

The presence of the ducts 16, however, means that the part of the container 3 above the free surface of the liquid L is always at atmospheric pressure. In order to avoid the escape of boiling vapours to the exterior through the ducts 16, the dimensions of the ducts 16 themselves are selected so that the boiling vapours which enter them condense completely and fall back into the container 3.

The Applicants have found that, with the temperature of the condenser element 4 kept more or less constant and approximately equal to the ambient temperature, holes 16 having a diameter of about 2 mm and a length (with reference straight holes) of about 40 mm are able to ensure complete condensation of the vapour which penetrates them, thus avoiding loss to the exterior.

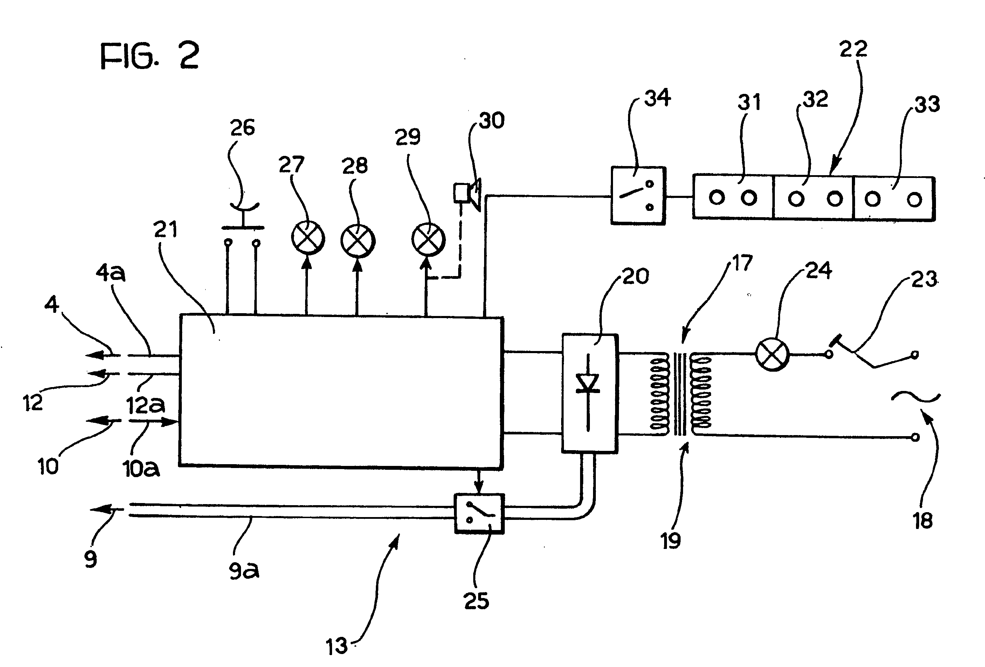

The control and operating circuit 13, the structure of which is illustrated in block schematic form in Figure 2, is constituted essentially by :

– a supply network 17 connected to the supply lead 18 of the apparatus and including a transformer 19 and a rectifier block 20,

– a processing unit 21 constituted essentially by a microprocessor, and

– a visual display unit 22 connected to the microprocessor 21.

In the primary circuit of the transformer 19 are connected a switch 23 constituting the main operating switch of the apparatus, and a first indicator light 24 which indicates that the switch 23 is closed and the apparatus is switched on. The supply network 17 is connected to the resistor 9 for heating the liquid L in the container 3 through a switch 25 which, to advantage, may be constituted by a solid-state switch controlled by the unit 21.

The unit 21 itself is connected through interfacing elements, not explicitly shown, to connection lines, 10a, 12a, and 4a respectively, to the temperature sensor 10, the electrode 12 and the body of the condenser element 4, of electrically conductive material. The arrangement of the connection of the electrode 12 is such that the electrode 12 itself, the condenser element 4 and their connecting cables 4a and 12a connecting them to the unit 21 form an electrical circuit which is normally open as a result of the presence of an air space (gap) between the vertex portion 6 of the condenser element 4 and the electrode 12. The width of this space or gap may be varied selectively in dependence on the specific applicational requirements by acting on adjustment members (not illustrated explicitly) associated with the electrodes 12.

This normally-open circuit may be closed only as a result of the establishment of an electrically conductive bridge between the vertex 6 and the electrode 12.

A push-button 26 allows an operator to start the measuring cycle. A further lamp 27, also connected to the unit 21, is arranged to indicate whether the test is in progress, while another lamp 28 indicates, according to criteria which will be explained more fully below, a momentary delay of the start of the test.

Finally, an optical warning device 29, which may be replaced by or assisted by an acoustic warning device 30, is provided to indicate the completion of the test to the operators.

The visual display unit 22 is arranged to allow the temperature signal generated by the sensor 10 to be presented to the exterior.

In the embodiment illustrated, the visual display unit 22 is constituted essentially by three pairs of indicators (lamps or LEDs) 31, 32 and 33, each of which is activated when the signal generated by the sensor 10 falls within a particular temperature range. For example, the lighting of the indicators 31 may correspond to a measured temperature within the range 100 to 125°C, the lighting of the indicators 32 may correspond to a temperature of between 125 and 140°C, while the measurement of a temperature above 140°C causes the lighting of the indicators 33.

A selector 34 enables the three temperature ranges identified by the lighting of the indicators 31, 32 and 33 to be varied, these being "slid" upwardly or downwardly and possibly changed in width.

For example, the selector switch 34 may be constituted by a two-position switch (shown by the respective terms DOT 3 and DOT 4 indicative of the corresponding standards with regard to the characteristics of the fluids used in motor vehicle brake systems) which can move the temperature ranges corresponding to the activation of the indicators 31, 32, 33 from a lower temperature range (DOT 3) to a higher temperature range (DOT 4).

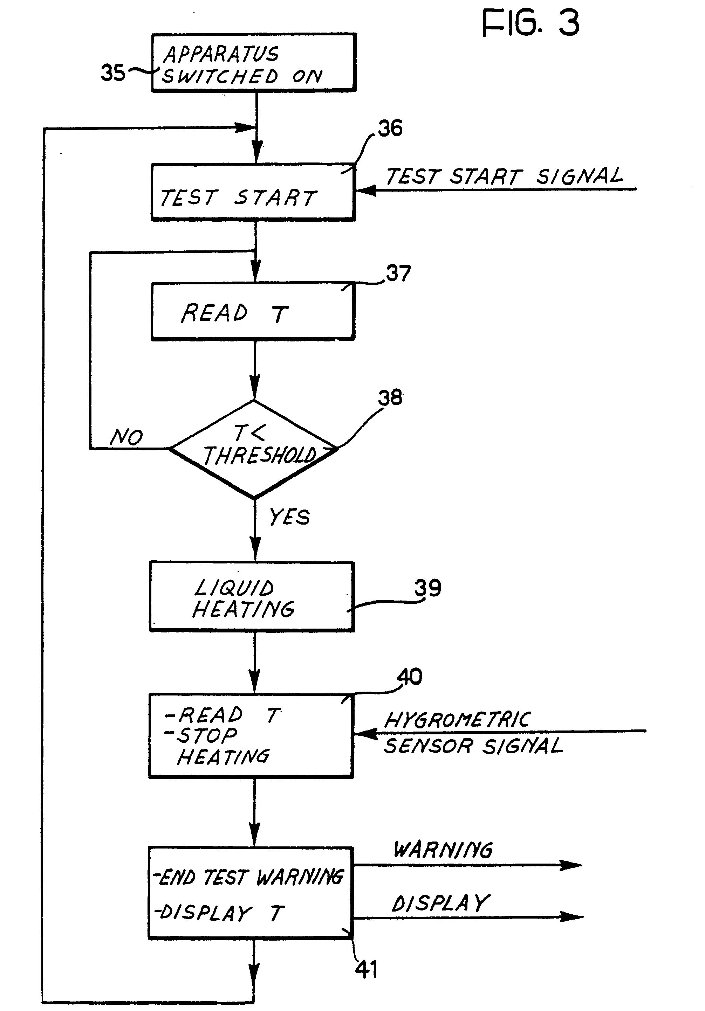

The processing unit (microprocessor 21) is programmed (according to generally known criteria which will not be explained in detail in that they are not essential for an understanding of the invention) in order to achieve the sequence of use which will now be described briefly with reference to the flow diagram of Figure 3.

As a preliminary operation, the operator will take up a predetermined quantity of the liquid whose boiling point it is wished to detect, for example with a syringe, and introduce it into the container 3. After the introduction of the liquid L, the container 3 is closed at the top by the application of the condenser element 4 so as to close the mouth of the container 3 itself.

By closing the switch 23, the operator connects the transformer 17 to the electrical network to which the lead 18 is connected, whereby the microprocessor 21 is supplied and arranged to start the test. The switching on of the apparatus (step 35 in the diagram of Figure 3) is indicated by the lighting up of the indicator lamp 24.

Subsequently, by pressing the push-button 26, the operator sends a test start signal to the microprocessor 21 (step 36).

The microprocessor detects the signal generated by the temperature sensor 10 (step 37) and compares it with a threshold corresponding to a lower temperature threshold, selected at around 50° (step 38).

If the temperature of the liquid is higher than this level, which could falsify the result of the test by altering the heating gradient in the liquid, the test procedure is momentarily interrupted to be restarted only when it is ascertained, after one or more succesive temperature readings, that the temperature of the liquid has fallen below the threshold.

At this point, the microprocessor 21 closes the switch to activate the resistor 9 and start the heating of the liquid L in the container 3 (step 39).

The heating of the liquid continues for a period of about 5 to 10 minutes until the liquid L reaches boiling point. In fact, the resistor 9 is normally low-powered (for example, of the order of 60W) to take account of the fact that the apparatus 1 is preferably portable.

The vapours which are evolved from the surface of the liquid rise towards the condenser element 4, condensing on the surface of the lower part 5 of the condenser element 4 itself.

As described above, the pressure in the space between the surface of the free film of the liquid L and the condenser element 4 is kept at about atmospheric since this space communicates with the exterior through the ducts 16. At the same time, however, the vapours which enter these ducts condense within the ducts themselves and fall back into the container 3. There is thus no loss of vapour to the exterior.

The vapour which condenses on the surface of the lower part 5 of the element 4 flows under gravity towards the vertex 6 until it forms a first drop of condensation. Immediately it has formed, the drop establishes a conductive bridge between the vertex 6 and the electrode 12.

The electrical circuit constituted by the condenser element 4, the electrode 12 and their connecting ducts 4a and 12a is closed to cause the emission of a corresponding signal to the microprocessor 21. In other words, this circuit behaves as a hygrometric sensor which indicates to the microprocessor the fact that the liquid L has reached boiling point.

Upon receipt of the signal from the hygrometric sensor (step 40 in the diagram of Figure 3), the microprocessor 21 notes the signal generated by the temperature sensor 10, simultaneously opening the switch 25 which controls the supply to the resistor 9 so as to stop the heating of the liquid L.

The signal generated by the temperature sensor 10, which is indicative of the temperature of the liquid L at the boiling point, is transmitted to the visual display unit (step 41), causing the lighting of one of the pairs of indicators 31, 32 or 33 in dependence on the temperature value.

At the same time, by activating the optical warning device 29 and/or the acoustic warning device 30, the microprocessor 21 signals to the operator that the test is completed, indicating that the data corresponding to the boiling point determined is being presented on the visual display unit 22.

In general, the lighting of the visual display elements 33 corresponds to the detection of a high boiling point which, in the case of a liquid for a braking system, indicates that the liquid can be considered as perfectly suitable for use and does not require replacement.

The lighting of the elements 32 is indicative of a certain lowering of the boiling point of the liquid, which advises its replacement in the medium-short term.

The activation of the elements 31, however, is indicative of a low boiling point. This indication corresponds to the need to replace the liquid quickly, it not being suitable for carrying out its function.

## Claims

1. Apparatus for determining the boiling point of liquids, comprising :
- a container (3) which can receive a predetermined quantity of the liquid (L) whose boiling point it is wished to determine,
- heating means (9) associated with the container (3), and
- a temperature sensor(10)sensitive to the temperature of the liquid (L) in the container and able to supply a thermometric signal (10a) indicative of this temperature, characterised in that it comprises :
- a condenser element (4) superposed on the container (3), which is able to condense the vapours from the boiling liquid (L) in the container and has at least one collecting point (6) for the drops of condensation,
- a hygrometric sensor (12) associated with the condenser element (4) and able to generate a reading signal as a result of the formation of one drop of condensation at the collecting point (6), and
- an electrical control and operating circuit (13) connected to the heating means (9), to the temperature sensor (10) and to the hygrometric sensor (12), and able to cause, in sequence, the activation of the heating means (9) and, upon the emission by the hygrometric sensor (12) of the reading signal corresponding to the formation of the first drop of condensation at the collecting point (6), the presentation to the exterior (22) of the thermometric signal emitted by the temperature sensor (10) at that moment.

2. Apparatus according to Claim 1, characterised in that the electronic control and operating circuit (13) is arranged so as to deactivate the heating means (9) upon the emission of the reading signal by the hygrometric sensor (12).

3. Apparatus according to Claim 2, characterised in that the electronic control and operating circuit (13) is arranged so that, after the deactivation, the heating means (9) are not subsequently reactivated until the temperature of the liquid (L) in the container (3) remains below a lower heating threshold (38).

4. Apparatus according to any one of Claims 1 to 3, characterised in that the condensing element (4) has a generally pointed part (5) facing the container (3), the vertex (6) of which defines the collecting point (6) for the drops of condensation.

5. Apparatus according to Claim 4, characterised in that the generally pointed part (5) is generally cone-shaped.

6. Apparatus according to any one of Claims 1 to 5, characterised in that the condensing element (4) is fitted so as to close the container (3) in a substantially sealed condition (8) with respect to the boiling vapours.

7. Apparatus according to Claim 6, characterised in that the condenser element (4) has ducts for putting the container (3) into communication with the external environment, the dimensions of the ducts (16) being selected so that the vapours from the boiling liquid (L) in the container which enter the ducts (16) condense completely within the ducts (16) themselves.

8. Apparatus according to any one of the preceding claims, characterised in that the temperature sensor (10) has a heat-sensitive member (11) which, in use, is located in the container (3) at a level equal to about half the level of filling of the liquid (L) whose boiling point it is wished to determine.

9. Apparatus according to any one of Claims 1 to 8, characterised in that the electrical control and operating circuit (13) has an associated visual display unit (22) including several indicator elements (31, 32, 33), each of which is intended to present to the exterior a thermometric signal (10a) corresponding to a temperature of the liquid (L) in the container falling within a respective predetermined range of temperatures.

10. Apparatus according to Claim 9, characterised in that the visual display unit (22) has associated selector means (34) for enabling the respective predetermined temperature ranges associated with each of the visual display elements (31, 32, 33) to be varied selectively.

11. Apparatus according to any of Claims 1 to 10, characterised in that said electrical control and operating circuit (13) is constituted, at least partly, by an electronic programmable circuit (21).

12. Method for determining the boiling point of liquids, comprising the step of heating a predetermined quantity of the liquid (L) whose boiling point it is wished to determine, characterised in that it further comprises the steps of :
- collecting the vapours from the boiling liquid (L) so as to form drops of condensation (6) from the vapours,
- detecting (12) the formation of the first drop of condensation, and
- measuring (10) the temperature of the liquid (L) on formation of the first drop of condensation.

## Ansprüche

1. Vorrichtung, um den Siedepunkt von Flüssigkeiten zu bestimmen, wobei die Vorrichtung enthält :
- einen Behälter (3), der eine vorgegebene Menge der Flüssigkeit (L) aufnehmen kann, deren Siedepunkt bestimmt werden soll,
- eine Heizeinrichtung (9), die dem Behälter (3) zugeordnet ist, und
– einen Temperaturfühler (10), der auf die Temperatur der Flüssigkeit (L) im Behälter anspricht und ein thermometrisches Signal (10a) liefern kann, das diese Temperatur anzeigt, dadurch gekennzeichnet, daß die Vorrichtung enthält :
– ein Kondensatorelement (4), das über dem Behälter (3) liegt und die Dämpfe der siedenden Flüssigkeit (L) im Behälter kondensieren kann, wobei es zumindest einen Sammelpunkt (6) für die Kondensationstropfen besitzt,
– einen Feuchtigkeitsmeßfühler (12), der dem Kondensatorelement (4) zugeordnet ist und ein Ablesesignal erzeugen kann, wenn am Sammelpunkt (6) ein Kondensationstropfen ausgebildet wird, und
– eine elektrische Steuer- und Betriebsstufe (13), die mit der Heizeinrichtung (9), dem Temperaturfühler (10) und dem Feuchtigkeitsmeßfühler (12) verbunden ist und nacheinander die Inbetriebsetzung der Heizeinrichtung (9) sowie dann, wenn der Feuchtigkeitsmeßfühler (12) jenes Signal abgibt, das der Ausbildung des ersten Kondensationstropfens am Sammelpunkt (6) entspricht, die Darstellung des thermometrischen Signals nach außen (22) veranlassen kann, das in diesem Augenblick vom Temperaturfühler (10) abgegeben wird.

2. Vorrichtung gemäß Anspruch 1, dadurch gekennzeichnet, daß die-elektronische steuer- und Betriebsstufe (13) so aufgebaut ist, um die Heizeinrichtung (9) bei der Abgabe des Ablesesignals durch den Feuchtigkeitsmeßfühler (12) außer Betrieb zu setzen.

3. Vorrichtung gemäß Anspruch 2, dadurch gekennzeichnet, daß die elektronische steuer- und Betriebsstufe (13) so aufgebaut ist, daß nach der Außerbetriebsetzung der Heizeinrichtung (9) diese nicht mehr in Betrieb gesetzt wird, bis die Temperatur der Flüssigkeit (L) im Behälter (3) unterhalb eines unteren Heizschwellwerts (38) bleibt.

4. Vorrichtung gemäß jedem der Ansprüche 1 bis 3, dadurch gekennzeichnet, daß das Kondensatorelement (4) einen im allgemeinen spitzen Teil (5) besitzt, der dem Behälter (3) gegenüberliegt, wobei dessen Spitze (6) den sammelpunkt (6) für die Kondensationstropfen bildet.

5. Vorrichtung gemäß Anspruch 4, dadurch gekennzeichnet, daß der im allgemeinen spitze Teil (5) im allgemeinen kegelförmig ausgebildet ist.

6. Vorrichtung gemäß jedem der Ansprüche 1 bis 5, dadurch gekennzeichnet, daß das Kondensatorelement (4) so nahe wie möglich beim Behälter (3) in einem im wesentlichen abgedichteten Zustand (8) im Hinblick auf die Siededämpfe sitzt.

7. Vorrichtung gemäß Anspruch 6, dadurch gekennzeichnet, daß das Kondensatorelement (4) Kanäle besitzt, um den Behälter (3) mit der äußeren Umgebung in Verbindung zu bringen, wobei die Abmessung der Kanäle (16) so gewählt werden, daß die Dämpfe von der siedenden Flüssigkeit (L) im Behälter, die in die Kanäle (16) eindringen, vollkommen innerhalb der Kanäle (16) selbst kondensieren.

8. Vorrichtung gemäß jedem der bisherigen Ansprüche, dadurch gekennzeichnet, daß der Temperaturfühler (10) einen wärmeempfindlichen Bauteil (11) besitzt, der im Betrieb im Behälter (3) auf einem Niveau angeordnet wird, das etwas dem halben Füllniveau der Flüssigkeit (L) entspricht, deren Siedepunkt bestimmt werden soll.

9. Vorrichtung gemäß jedem der Ansprüche 1 bis 8, dadurch gekennzeichnet, daß die elektrische steuer- und Betriebsstufe (13) eine zugeordnete optische Anzeigeeinheit (22) besitzt, die mehrere Anzeigeelemente (31, 32, 33) aufweist, von denen jedes vorgesehen ist, um nach außen ein thermometrisches Signal (10a) abzugeben, das einer Temperatur der Flüssigkeit (L) im Behälter entspricht, die in einem entsprechenden vorgegebenen Temperaturbereich liegt.

10. Vorrichtung gemäß Anspruch 9, dadurch gekennzeichnet, daß der optischen Anzeigeeinheit (22) eine Auswahleinrichtung (34) zugeordnet ist, um die entsprechenden vorgegebenen Temperaturbereiche wahlweise verändern zu können, die jedem optischen Anzeigeelement (31, 32, 33) zugeordnet sind.

11. Vorrichtung gemäß jedem der Ansprüche 1 bis 10, dadurch gekennzeichnet, daß die elektrische steuer- und Betriebsstufe (13) zumindest teilweise von einem programmierbaren Elektronikschaltkreis (21) gebildet wird.

12. Verfahren, um den Siedepunkt von Flüssigkeiten zu bestimmen, wobei das Verfahren folgenden schritt enthält :
– Erhitzen einer vorgegebenen Menge der Flüssigkeit (L), deren Siedepunkt bestimmt werden soll, dadurch gekennzeichnet, daß das Verfahren weiters folgende schritte enthält :
– sammeln der Dämpfe von der siedenden Flüssigkeit (L), um Kondensationstropfen (6) von dem Dämpfen zu bilden,
– Ermitteln (12) der Ausbildung des ersten Kondensationstropfens und
– Messen (10) der Temperatur der Flüssigkeit (L) beim Ausbilden des ersten Kondensationstropfens.

## Revendications

1. Appareil pour déterminer le point d'ébullition de liquides, comprenant :
– un récipient (3) qui peut recevoir une quantité prédéterminée de liquide (L) dont on souhaite déterminer le point d'ébullition,
– un moyen de chauffage (9) associé au récipient (3), et

– un capteur de température (10) sensible à la température du liquide (L) contenu dans le récipient et capable de produire un signal thermométrique (10a) qui constitue une indication de cette température, caractérisé en ce qu'il comprend :

– un élément formant condenseur (4) disposé sur le récipient (3), qui est capable de condenser les vapeurs du liquide (L) en ébullition dans le récipient et qui comporte au moins un point de collecte (6) pour les gouttes de condensation,

– un capteur hygrométrique (12) associé à l'élément formant condenseur (4) et capable de produire un signal de lecture par suite de la formation d'une goutte de condensation au point de collecte (6), et

– un circuit électrique de commande et d'actionnement (13) relié au moyen de chauffage (9), au capteur de température (10) et au capteur hygrométrique (12), et capable de provoquer, successivement, l'activation du moyen de chauffage (9) et, lors de l'émission par le capteur hygrométrique (12) du signal de lecture correspondant à la formation de la première goutte de condensation au point de collecte (6), la présentation à l'extérieur (22) du signal thermométrique émis par le capteur de température (10) à ce moment.

2. Appareil selon la revendication 1, caractérisé en ce que le circuit électronique de commande et d'actionnement (13) est agencé de façon à désactiver le moyen de chauffage (9) lors de l'émission du signal de lecture par le capteur hygrométrique (12).

3. Appareil selon la revendication 2, caractérisé en ce que le circuit électronique de commande et d'actionnement (13) est agencé de façon que, après la désactivation, le moyen de chauffage (9) ne soit plus réactivé ultérieurement jusqu'à ce que la température du liquide (L) dans le récipient (3) demeure inférieure à un seuil inférieur de chauffage (38).

4. Appareil selon l'une quelconque des revendications 1 à 3, caractérisé en ce que l'élément de condensation (4) comporte une partie (5) généralement en pointe tournée vers le récipient (3), dont le sommet (6) définit le point de collecte (6) pour les gouttes de condensation.

5. Appareil selon la revendication 4, caractérisé en ce que la partie (5) généralement en pointe a la forme générale d'un cône.

6. Appareil selon l'une quelconque des revendications 1 à 5, caractérisé en ce que l'élément de condensation (4) est disposé de façon à fermer le récipient (3) dans un état (8) sensiblement hermétique vis-à-vis des vapeurs d'ébullition.

7. Appareil selon la revendication 6, caractérisé en ce que l'élément formant condenseur (4) comporte des conduites pour mettre le récipient (3) en communication avec l'environnement externe, les dimensions des conduites (16) étant choisies de façon que les vapeurs provenant du liquide (L) en ébullition dans le récipient qui pénètrent dans les conduites (16) se condensent complètement à l'intérieur des conduites (16) elles-mêmes.

8. Appareil selon l'une quelconque des revendications précédentes, caractérisé en ce que le capteur de température (10) comporte un élément thermosensible (11) qui, lors de l'utilisation, est situé dans le récipient (3) à un niveau égal à environ la moitié du niveau de remplissage du liquide (L) dont on souhaite déterminer le point d'ébullition.

9. Appareil selon l'une quelconque des revendications 1 à 8, caractérisé en ce que le circuit électrique de commande et d'actionnement (13) comporte une unité d'affichage visuel (22) associée comprenant plusieurs éléments indicateurs (31, 32, 33) destinés chacun à présenter à l'extérieur un signal thermométrique (10a) correspondant à une température du liquide (L) dans le récipient située dans une gamme de température prédéterminée respective.

10. Appareil selon la revendication 9, caractérisé en ce que l'unité d'affichage visuel (22) comporte un moyen formant sélecteur (34) associé pour permettre de modifier sélectivement les gammes de température prédéterminées respectives associées à chacun des éléments d'affichage visuel (31, 32, 33).

11. Appareil selon l'une quelconque des revendications 1 à 10, caractérisé en ce que ledit circuit électrique de commande et d'actionnement (13) est constitué, au moins partiellement, par un circuit électronique programmable (21).

12. Procédé de détermination du point d'ébullition de liquides, comprenant l'étape de chauffage d'une quantité prédéterminée du liquide (L) dont on souhaite déterminer le point d'ébullition, caractérisé en ce qu'il comprend en outre les étapes suivantes :

– collecte des vapeurs provenant du liquide (L) en ébullition de façon à former des gouttes de condensation (6) à partir des vapeurs,

– détection (12) de la formation de la première goutte de condensation, et

– mesure (10) de la température du liquide (L) lors de la formation de la première goutte de condensation .

FIG. 1

FIG. 2

# FIG. 3

Flowchart:

35 — APPARATUS SWITCHED ON

36 — TEST START ← TEST START SIGNAL

37 — READ T

38 — T < THRESHOLD? — NO / YES

39 — LIQUID HEATING

40 — -READ T / -STOP HEATING ← HYGROMETRIC SENSOR SIGNAL

41 — -END TEST WARNING / -DISPLAY T → WARNING / DISPLAY